# EUROPEAN PATENT APPLICATION

(11) **EP 1 649 878 A2**
(43) Date of publication of application: **26.04.2006**
(21) Application number: 05021160.6
(22) Date of filing: 28.09.2005
(51) Int. Cl.: A61L 27/38, A61L 27/12

(54) **Method of manufacturing globular ceramic particles, method of seeding cells and method of manufacturing product for repairing biological tissues**

(30) Priority: 01.10.2004 JP 2004290224
(71) Applicant: Olympus Corporation, Tokyo 151-0072 (JP)
(72) Inventor: Sadamori, Katsuya, Hino-shi Tokyo 151-0072 (JP); Shiba, Yoshiaki, Hachioji-shi Tokyo 192-0023 (JP)
(74) Representative: von Hellfeld, Axel

(57) **Abstract**

To efficiently manufacture globular ceramic particles which can be formed uniformly in surrounding gaps when they are arbitrarily filled into a defect part of biological tissue or the like, without impairing the adhesiveness of cells. There is provided a method of manufacturing globular ceramic particles comprising; containing a predetermined amount of granular shaped ceramic particles inside a cylindrical container 5 that is arranged approximately horizontally, and rotating the cylindrical container about its axis.

## Description

### BACKGROUND OF THE INVENTION

### 1. FIELD OF THE INVENTION

The present invention relates to a method of manufacturing globular ceramic particles, a method of seeding cells, and a method of manufacturing a product for repairing biological tissues.

This application is based on patent application No. 2004-290224 filed in Japan, the content of which is incorporated herein by reference.

### 2. DESCRIPTION OF RELATED ART

Recently, it has become possible to repair a defect part of bone caused by osteoncus extraction, trauma, or the like, by regenerating the bone by filling a bone-repairing material. For such a bone-repairing material, hydroxyapatite (HAP) and tricalcium phosphate (TCP) are known. However, from the viewpoint of leaving no foreign matter inside the body, a scaffold made of a porous calcium phosphate material, for example, such as β-TCP is used. If the β-TCP is left in contact with bone cells of a defect part of bone, so-called remodeling is performed in which osteoclasts absorb the β-TCP, and osteoblasts form a new bone. That is, the bone-repairing material filled in the defect part of the bone is replaced by autologous bone as time goes by (for example, refer to: Uemura and two others, "Tissue engineering in bone using biodegradable β-TCP porous material - A new material strengthened in vivo Osferion", Medical Asahi, The Asahi Shimbun Company, October 1, 2001, Vol. 30, No. 10, p. 46-49).

The shape of a bone-repairing material is generally known to be a cubic block shape, a granular shape, and the like. The shape of a bone-repairing material can be properly selected according to the form of a defect part of bone. If a defect part of bone covers a relatively wide-range, firstly the majority of the volume is filled with larger cubic block shaped bone-repairing material. Next, smaller cubic block shaped bone-repairing material is filled in the gaps, and then granular shaped bone-repairing material is filled in the even smaller gaps.

### Disclosure of Invention

If the bone-repairing material is powder, the overall internal volume can be filled in a high density without gaps, so as to fit with complex shapes peculiar to various defect parts of bone. However, since the gaps are completely filled with the powder, a route for cells to grow, a route for supplying nutrients to the cells, and a route for discharging waste matters from the cells can not be maintained. Therefore, a cubic block shape or granular shape porous calcium phosphate material is used. However, since a cubic block shape bone-repairing product is angular, there is a disadvantage in that nonuniform gaps are formed in the surroundings, unless the filling is done in an orderly manner. Moreover, since each granular body of the granular shaped bone-repairing material has an irregular shape, gaps between the granular bodies easily become nonuniform, although this is not as bad as for the case of cubic block shaped bone-repairing product. Similarly in a method in which gaps between the cubic block shaped bone-repairing products are filled with granular shaped bone-repairing material, large gaps and small gaps also occur.

If in this manner nonuniform gaps are formed in the surroundings of the material for repairing biological tissues such as bone-repairing material, the following disadvantages can be considered.

Firstly, as mentioned above, if nonuniform gaps are formed in the surroundings of the material for repairing biological tissues, then in a state where the material is filled into a defect part of the biological tissue, the flow of the body fluid in the surroundings becomes nonuniform. As a result, the density of cells that grow using the material for repairing biological tissues as a scaffold becomes nonuniform, and hence there is a disadvantage in that the biological tissue is not uniformly regenerated.

Secondly, if nonuniform gaps are formed in the surroundings of the material for repairing biological tissues that has been filled into the internal space of a bioreactor such as an artificial liver, the function as the bioreactor can not be realized all over the internal space, and hence there is a disadvantage in that high performance can not be obtained.

Thirdly, if the material for repairing biological tissues is put into a medium together with cells and cultured, in a state with nonuniform gaps formed in the above manner, the cell distribution becomes nonuniform, and hence there is a disadvantage in that accurate judgement can not be made in the shipping inspection and the like. That is, a situation may arise where, since a relatively large number of cells were present in a sample collected in the shipping inspection, then a shipping judgement is made, but in fact in another parts no cells were present at all. Conversely, another situation may arise where, since no cells were present in a sample, then culturing was continued, but in fact cells were sufficiently proliferated in another parts. In order to avoid such disadvantages, samples should be collected from at least two parts in performing the inspection. However there is then the disadvantage in that the number of the work steps are increased.

### BRIEF SUMMARY OF THE INVENTION

The present invention takes the above problems into consideration, with an object of providing: a method of manufacturing globular ceramic particles which can be formed uniformly in the surrounding gaps, when they are arbitrarily filled into a defect part of biological tissue or the like, without impairing the adhesiveness of cells; a method of seeding cells whereby cells can be efficiently seeded; and a method of manufacturing a product for repairing biological tissues whereby the product for repairing biological tissues can be efficiently manufactured.

In order to solve the above problem, the present invention employs the following solutions.

The present invention provides a method of manufacturing globular ceramic particles comprising; containing a predetermined amount of ceramic particles inside a cylindrical container that is arranged approximately horizontally, and rotating the cylindrical container about its axis.

According to the present invention, by rotating the cylindrical container containing ceramic particles about its horizontal axis, movement is repeated inside the cylindrical container where the ceramic particles are lifted up to a predetermined height together with the rotation of the cylindrical container, and then dropped by the gravity. As a result, the ceramic particles are abraded due to friction and collision generated between the ceramic particles or between the ceramic particles and the internal surface of the cylindrical container, and thus formed into globular shapes with time. Therefore, it is possible to readily manufacture globular ceramic particles which can be formed uniformly into the surrounding gaps when they are arbitrarily filled into a defect part of biological tissue or the like, without impairing the adhesiveness of cells.

Moreover, the present invention provides a method of seeding cells comprising; containing globular ceramic particles inside a cylindrical container that is arranged approximately horizontally, enclosing a cell suspension having suspended cells to be seeded inside the cylindrical container, and then rotating the cylindrical container about its axis.

According to the present invention, the globular ceramic particles and the cell suspension are enclosed inside the cylindrical container, and the cylindrical container is rotated about its horizontal axis, to thereby make the globular ceramic particles contact with the cell suspension, so that they are adhered to the internal surface of the cylindrical container due to surface tension. Then as the cylindrical container is being rotated, the globular ceramic particles adhered to the internal surface of the cylindrical container are made to pass through the cell suspension that has been pooled at the bottom. Since the cells to be seeded are suspended in the cell suspension, the cells are captured by the globular ceramic particles passing through the cell suspension, and seeded. As a result, the cells can be efficiently seeded on the globular ceramic particles.

In the above structure, preferably the amount of the globular ceramic particles is such that, if the globular ceramic particles are laid over the cylindrical internal surface of the cylindrical container in one layer, almost all of the cylindrical internal surface is covered.

By such a structure, if the globular ceramic particles are adhered to the cylindrical internal surface of the cylindrical container in one layer, almost all of the cylindrical internal surface is covered and the globular ceramic particles are continually soaked in the cell suspension. In that case, since the globular ceramic particles pass in the vicinity of the bottom of the cell suspension which is pooled at the bottom of the cylindrical container, the cells which tend to subside into the cell suspension are efficiently seeded on the globular ceramic particles.

Moreover, the present invention provides a method of manufacturing a product for repairing biological tissues, comprising: a step for manufacturing globular ceramic particles comprising containing ceramic particles inside in a cylindrical container that is arranged approximately horizontally, and rotating the cylindrical container about its axis; and a seeding step comprising enclosing in the cylindrical container the manufactured globular ceramic particles and a cell suspension in which cells to adhere to the manufactured globular ceramic particles are suspended, and then rotating the cylindrical container about its axis.

According to the present invention, by rotating the cylindrical container containing ceramic particles about its horizontal axis, the ceramic particles are abraded, so that it becomes possible to readily manufacture globular ceramic particles which can be formed uniformly in the surrounding gaps, when they are arbitrarily filled into a defect part of biological tissue or the like, without impairing the adhesiveness of cells. Moreover, the cell suspension is enclosed in the cylindrical container which is again rotated again about its axis, so that the cells in the cell suspension are captured by the globular ceramic particles, and the cells are efficiently seeded, enabling manufacture of the product for repairing biological tissues.

In the above structure, preferably a washing step for washing out scrap powder that has adhered onto the globular ceramic particles, is provided between the manufacturing step and the seeding step.

By so doing, the scrap powder that has been generated from the abrasion of the granular shaped ceramic particles, and adhered onto the globular ceramic particles is washed out in the washing step, and the adhesiveness of the cells is increased, so that the product for repairing biological tissues can be manufactured more efficiently.

In the above structure, preferably the rotating speed of the cylindrical container in the manufacturing step is 20 to 100 rpm, and the rotating speed of the cylindrical container in the seeding step is 0.25 to 10 rpm.

By so doing, by simply changing the rotating speed, the globular ceramic particles can be manufactured, and the cells can be seeded onto the globular ceramic particles in the same or similar cylindrical containers, enabling manufacture of the product for repairing biological tissues. By setting the rotating speed in the manufacturing step to 20 to 100 rpm, the ceramic particles are moved so as to roll on the internal surface of the cylindrical container, enabling efficient manufacture of the globular ceramic particles product for repairing biological tissues. Moreover, by setting the rotating speed in the seeding step to 0.25 to 10 rpm, the globular ceramic particles that have adhered onto the internal surface of the cylindrical container by the cell suspension, can pass through the cell suspension while being adhered, enabling the cells to be seeded efficiently.

Furthermore, in the above structure, preferably the amount of globular ceramic particles is such that, if the globular ceramic particles are laid over the cylindrical internal surface of the cylindrical container in one layer, almost all of the cylindrical internal surface is covered.

By so doing, in a state where the globular ceramic particles are covering almost all of the cylindrical internal surface of the cylindrical container, they can be continually soaked in the cell suspension. Therefore the cells which tend to subside into the cell suspension can be efficiently seeded on the globular ceramic particles, and the product for repairing biological tissues can be manufactured efficiently.

According to the present invention, the following effects can be demonstrated. The angular parts of ceramic particles can be abraded so that the globular ceramic particles can be readily manufactured. Moreover the cells can be efficiently seeded on the manufactured globular ceramic particles, and the product for repairing biological tissues having the cells soaked into the inside, can be efficiently manufactured.

### BRIEF DESCRIPTION OF THE SEVERAL VIEWS OF THE DRAWINGS

FIG. 1 is a flowchart showing a method of manufacturing a product for repairing biological tissues according to an embodiment of the present invention.
FIG. 2 is a perspective view showing a manufacturing step in the method of manufacturing a product for repairing biological tissues of FIG. 1.
FIG. 3A is a process drawing showing the state of ceramic particles in the manufacturing step of FIG. 2.
FIG. 3B is a process drawing showing the state of ceramic particles in the manufacturing step of FIG. 2.
FIG. 3C is a process drawing showing the state of ceramic particles in the manufacturing step of FIG. 2.
FIG. 4A is a process drawing showing a seeding step in the method of manufacturing a product for repairing biological tissues of FIG. 1.
FIG. 4B is a process drawing showing a seeding step in the method of manufacturing a product for repairing biological tissues of FIG. 1.
FIG. 4C is a process drawing showing a seeding step in the method of manufacturing a product for repairing biological tissues of FIG. 1.
FIG. 4D is a process drawing showing a seeding step in the method of manufacturing a product for repairing biological tissues of FIG. 1.

### DETAILED DESCRIPTION OF THE INVENTION

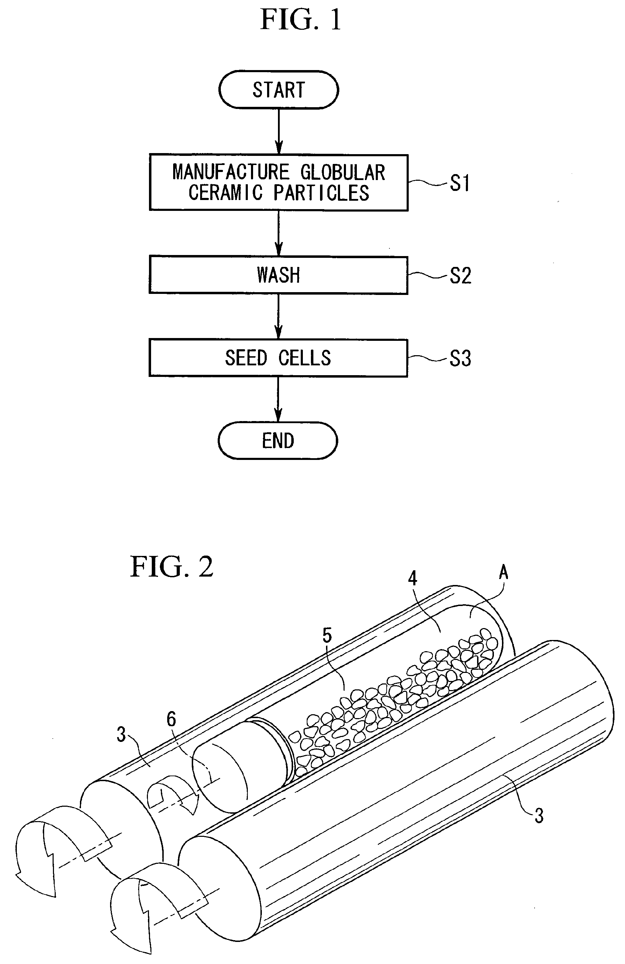

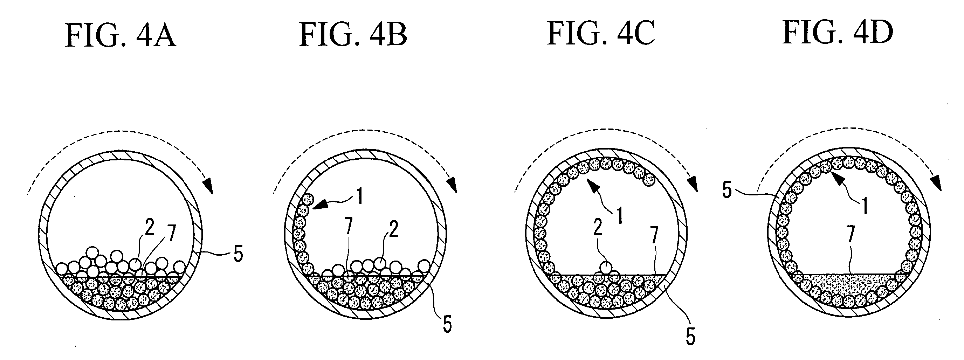

Hereunder is a description of a method of manufacturing a product 1 (refer to FIG. 4B,4C,4D) for repairing biological tissues according to an embodiment of the present invention, with reference to FIG. 1.

As shown in FIG.1, the method of manufacturing the product 1 for repairing biological tissues according to the present embodiment comprises: a step S1 for manufacturing globular ceramic particles 2; a washing step S2; and a step S3 for seeding cells onto the globular ceramic particles 2 manufactured in the manufacturing step S1.

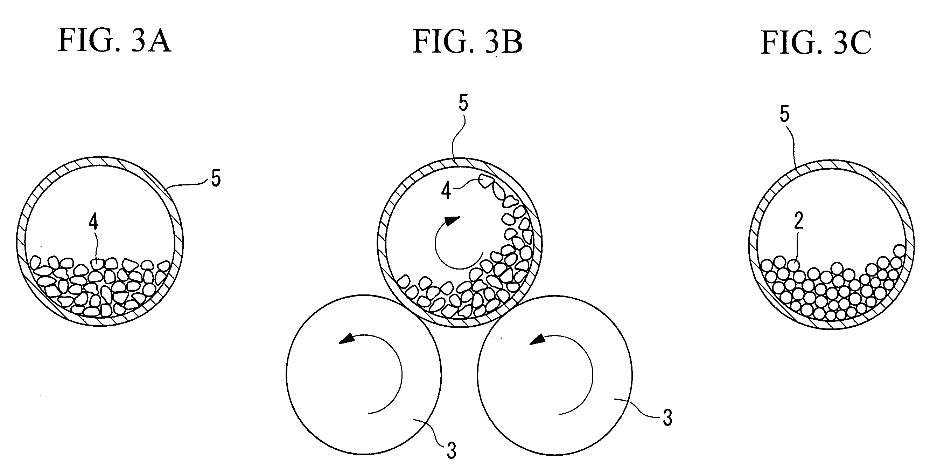

As shown in FIG. 2 and FIG. 3B, in the step S1 for manufacturing globular ceramic particles 2, a cylindrical container 5 containing granular shaped ceramic particles 4 is mounted on two approximately horizontal rollers 3 which have been arranged with a parallel spacing. Then by rotating the two rollers 3 in the same direction, the cylindrical container 5 is rotated about its horizontal central axis 6 (refer to FIG. 2). The rotating speed of the rollers 3 is about 20 to 100 rpm. For example, in the case of the 50 ml cylindrical container 5, the diameter is 27 mm. Therefore the rotating speed is set so that the peripheral speed becomes about 28 mm/s to 140 mm/s.

As shown in FIG. 3A, when the cylindrical container 5 is stationary, the granular shaped ceramic particles 4 inside of the cylindrical container 5 are accumulated at the bottom of the cylindrical container 5. As shown in FIG. 3B, by rotating the cylindrical container 5 within the above range of rotating speed, the granular shaped ceramic particles 4 inside of the cylindrical container are moved to roll on the internal surface of the cylindrical container 5. As a result, they are abraded due to friction and collision between the granules or between the granules and the internal surface of the cylindrical container 5, so that as shown in FIG. 3C, the globular ceramic particles 2 can be efficiently obtained.

The washing step S2 is a step for washing out the scrap powder from the ceramic particles 2 formed in the manufacturing step S1. In this step, for example optional cleaning solution such as pure water or physiological saline solution is introduced into the cylindrical container 5, and shaken, and then poured out from the cylindrical container 5. As a result, the scrap powder accumulated in the cylindrical container and adhered onto the surface of the globular ceramic particles 2 can be washed out.

In the seeding step S3, a cell suspension 7 (refer to FIG. 4A ) having suspended cells to be seeded is introduced into the cylindrical container 5 containing the globular ceramic particles 2 that have been washed in the washing step S2. Then it is rotated about its horizontal central axis 6 on the rollers 3. The rotating speed of the rollers 3 is about 0.25 to 10 rpm. For example, in the case of the 50 ml cylindrical container 5, the rotating speed is set so that the peripheral speed is within a range of 0.35 mm/s to 14mm/s.

As shown in FIG. 4A, in the seeding step S3, the cell suspension 7 to be introduced into the cylindrical container 5, is introduced in an amount such that it is pooled to a maximum depth deeper than the diameter of the globular ceramic particles 2 at the bottom inside the horizontally arranged cylindrical container 5. For example, in the case of the 50 ml cylindrical container 5, the amount is about 5 to 15ml.

Moreover, the amount of the globular ceramic particles 2 in the cylindrical container 5 is preferably such that if the globular ceramic particles 2 are laid over the internal surface of the cylindrical container 5, they are filled up over the entire internal surface of the cylindrical container 5 in one layer. For example, in the case of the 50 ml cylindrical container 5, the amount is about 3g.

When the cylindrical container 5 having the globular ceramic particles 2 and the cell suspension 7 enclosed in this manner is made horizontal and rotated about its central axis 6, the respective globular ceramic particles 2 are adhered onto the internal surface of the cylindrical container 5 due to the surface tension of the cell suspension 7, and as shown in FIG. 4B, are moved together with the rotation of the cylindrical container 5. As shown in FIG. 4C, the globular ceramic particles 2 that have been aggregated at the bottom of the cylindrical container 5 are sequentially adhered onto the internal surface of the cylindrical container 5 together with the rotation of the cylindrical container 5. Finally as shown in FIG. 4D, they are spread in one layer so as to fill up over the entire internal surface of the cylindrical container 5.

By further rotating the cylindrical container 5 in this state, the globular ceramic particles 2 that have been adhered onto the internal surface of the cylindrical container 5 are sequentially soaked in the cell suspension 7 pooled in the cylindrical container 5, together with the rotation of the cylindrical container 5. Since the cell suspension 7 in the cylindrical container 5 is pooled such that its maximum depth becomes deeper than the diameter of the globular ceramic particle 2, the globular ceramic particle 2 are soaked completely immersed in the cell suspension 7.

Moreover, since the globular ceramic particles 2 are moved while being adhered onto the internal surface of the cylindrical container 5, they are made to pass through the bottom of the cell suspension 7. Since the cells in the cell suspension 7 tend to subside due to the action of gravity, most of them are present at the bottom of the cell suspension 7. Consequently, by making the globular ceramic particles 2 pass through the bottom of the cell suspension 7, the globular ceramic particles 2 pass through the area where most of the cells are present, so that many cells can be captured.

By rotating the cylindrical container 5 in this manner for about 24 hrs, the cells can be efficiently seeded on the globular ceramic particles 2.

In this manner, according to the method of manufacturing the product 1 for repairing biological tissues according to the present embodiment, it is possible to readily manufacture the product 1 for repairing biological tissues having cells adhered onto the globular ceramic particles 2, which can be formed uniformly in the surrounding gaps when it is arbitrarily filled into a defect part of biological tissue or the like.

Moreover, according to the method of manufacturing the product 1 for repairing biological tissues constituted in this manner, a higher seedion efficiency can be obtained compared to the case where cell suspension and the material for repairing biological tissues are put into a petri dish and left standing.

In the method of manufacturing the product 1 for repairing biological tissues according to the present embodiment, the washing step S2 is set between the manufacturing step S1 and the seeding step S3. However the washing step S2 is not always necessary. In particular, if the cylindrical container 5 is replaced in the manufacturing step S1 and in the seeding step S3, the washing step S2 is not required. The optimum amount of the granular shaped ceramic particles 4 at the time of manufacturing the globular ceramic particles 2, and the amount of the globular ceramic particles 2 for enabling seeding at maximum efficiency are considered to be different. Therefore, the amount of the ceramic particles 2, and the ceramic particles 4 treated in step S1 and step S2 may be different.

Moreover, for the cylindrical container 5, in order to be able to manufacture the globular ceramic particles 2 more efficiently in the step S1 for manufacturing the globular ceramic particles 2, a cylindrical container having an internal surface structure where the granular shaped ceramic particles 2 can be rotatively rolled to become globular may be employed. For example, a cylindrical container having projections on the internal surface may be employed. For the projections, for example projections having a height of about 100 µm are preferable.

Moreover, for the raw material of the globular ceramic particles 2, in addition to β-tricalcium phosphate, other optional ceramic particles such as hydroxyapatite or the like may be employed.

Furthermore, for the cells to be seeded, in addition to mesenchymal stem cells, other optional adhesive cells may be employed.

## Claims

1. A method of manufacturing globular ceramic particles, comprising; containing a predetermined amount of ceramic particles inside a cylindrical container that is arranged approximately horizontally, and rotating said cylindrical container about its axis.

2. A method of seeding cells comprising;
containing globular ceramic particles inside a cylindrical container that is arranged approximately horizontally,
enclosing a cell suspension having suspended cells to be seeded inside the cylindrical container, and then
rotating said cylindrical container about its axis.

3. A method of seeding cells according to claim 2, wherein an amount of said globular ceramic particles is such that, if the globular ceramic particles are laid over the cylindrical internal surface of said cylindrical container in one layer, almost all of said cylindrical internal surface is covered.

4. A method of manufacturing a product for repairing biological tissues, comprising:
a step for manufacturing globular ceramic particles comprising containing ceramic particles inside a cylindrical container that is arranged approximately horizontally, and rotating said cylindrical container about its axis; and
a seeding step comprising enclosing a cell suspension having suspended cells to be seeded together with said manufactured globular ceramic particles, inside a cylindrical container, and then rotating said cylindrical container about its axis.

5. A method of manufacturing a product for repairing biological tissues according to claim 4, wherein a washing step for washing out scrap powder that has adhered onto the globular ceramic particles, is provided between said manufacturing step and said seeding step.

6. A method of manufacturing a product for repairing biological tissues according to claim 4, wherein the rotating speed of the cylindrical container in said manufacturing step is 20 to 100 rpm, and the rotating speed of the cylindrical container in said seeding step is 0.25 to 10 rpm.

7. A method of manufacturing a product for repairing biological tissues according to claim 4, wherein the amount of globular ceramic particles is such that, if the globular ceramic particles are laid over the cylindrical internal surface of said cylindrical container in one layer, almost all of said cylindrical internal surface is covered.
